# EUROPEAN PATENT APPLICATION

(11) **EP 4 085 947 A1**
(43) Date of publication of application: **09.11.2022**
(21) Application number: 21171945.5
(22) Date of filing: 04.05.2021
(51) Int. Cl.: A61M 5/315, A61M 5/50, A61M 5/20

(54) **DRUG DELIVERY DEVICE WITH CODE READER**

(71) Applicant: Ypsomed AG, 3401 Burgdorf (CH)
(72) Inventor: Scheurer, Simon, 3006 Bern (CH); Brügger, Martin, 3065 Bolligen (CH); Hostettler, Patrick, 3415 Hasle (CH)

(57) **Abstract**

The present invention relates to a drug delivery device (1) for dispensing a liquid drug from a reservoir (10). The delivery device (1) comprising a plunger rod (90), a drive mechanism for moving the plunger rod (90) in a dispensing direction and in a filling direction. The device further comprises the reservoir (10) including a piston (15). The plunger rod (90) is connectable to the piston (15) to move the piston (15) both in the dispensing direction and in the filling direction. The delivery device (1) further comprises a code reader (130) for reading code information and a controller adapted to compare the read code information with predefined information and adapted to control the drive mechanism to block the piston rod (90) or to move the piston rod (90) in the filling direction to fill the reservoir (10) with liquid drug based on the comparison.

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a drug delivery device comprising a drive mechanism with plunger rod movable in a dispensing direction and in opposite direction and connectable to a piston of a reservoir. The piston is movable by the plunger rod in the dispensing direction and in the opposite direction.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a cartridge, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic unit or control unit embedded or integrated in the delivery device, or being part of an auxiliary or supplemental electronic module or add-on device detachably attached to the delivery device. The electronic unit monitors a drug delivery process, in order to proactively prevent false handling of the device and/or to keep track of the doses already applied, and generates data related to an instantaneous condition and/or use of the delivery device.

Despite careful handling and controlling a lot of medication errors can happen in a hospital environment but also at the user's home during self-medication. Such errors include the administration of an improper dose, the administration of the wrong medication and the use of the wrong route of administration. Medication errors stem from poor communication, misinterpreted handwriting, drug name confusion, drug concentration, inaccurate dosage calculation, ambiguous drug labeling and packaging, lack of employee knowledge and/or lack of patient understanding about a drug's directions. There have been several attempts to avoid medication errors by means of safety systems and safety rules. In particular, systems are known in the art that verify a drug with predefined criteria before use in order to prevent the dispensing of an inappropriate drug.

For example, WO06084464 A1 describes an injection device with several lock-levels. The device comprises a cartridge with coded information in form of a bar code or a RFID. A code reader of the device can read the cartridge code. A device control unit compares the read code information such as a type of insulin, an expiry date, a production date or a country code. If the information are not according to a predefined rule the device can lock specific function such as a display or an administration of the drug.

WO16115477 discloses an injection device with a scan assembly that can scan a barcode of a container inserted into the injection device. A transmitter of the scan assembly is able to transmit the information from the barcode to a remote electronic database. The electronic database is adapted to verify the information contained on the barcode and transmit a signal to the injection device that provides positive feedback for injection using the injection device. If the database does not send a positive feedback (correct medication) an injection with the injection device is prevented by an activated interlock of the device.

EP2526986 B1 discloses an electronic injection device comprising a syringe inside a housing. The syringe comprises a bar code affixed as identification code. The identification code includes the preparation name, preparation code, manufacturer's name, manufacture date, place of manufacture, lot number, expiration date, destination, and other such information. A microprocessor compares the information read from the identification code of the syringe with criteria stored in the memory and if it is an appropriate syringe the injection processing is performed. If it is determined that the syringe is not correct an error message is displayed on the display section of the device.

These prior art approaches focus on a verification and to a locking of a dispensing mechanism in particular a locking of motor that is adapted to move a plunger rod to administer the drug. However, often such a lock can be overruled by the user.

### DESCRIPTION OF THE INVENTION

It is an objective of the invention to provide an efficient and a reliable safety mechanism for a drug delivery device.

This objective is achieved by the drug delivery device and the method according to the independent claims. Preferred embodiments are evident from the dependent claims.

According to the invention a drug delivery device for dispensing a liquid drug from a reservoir (cartridge, carpule) comprises a plunger rod, a manual (user force operable) or automatic (motor driven) drive mechanism for moving the plunger rod in a dispensing direction (distal direction) and in a filling direction (proximal direction) opposite the dispensing direction. The drug delivery device further comprises the reservoir including a piston movable relative to a reservoir outlet to dispense the liquid from the reservoir or to fill the reservoir with liquid drug by aspiration. The plunger rod is connectable to the piston to move the piston in both the dispensing direction and the filling direction. The drug delivery device further comprises a code reader for reading a machine-readable code with code information provided outside of the drug delivery device and a controller adapted to compare the read code information with predefined information. The controller is configured to control the drive mechanism to block the piston rod (to prevent a movement of the piston rod) or to move the piston rod in the filling direction in order to fill the reservoir with liquid drug from a drug container (e.g. vial) based on the comparison of the read information with the predefined information.

The connection between the piston and the plunger rod allows to pull the piston in a proximal direction (opposite the dispensing end) by the plunger rod to fill the reservoir with drug from a vial or a drug container.

The controller may lock or unlock the plunger rod based on a verification or a match of the read information with predefined and previously stored or received information. If the read information correspond to, match or are in compliance with the predefined information the controller enables or unlocks drives the drive mechanism to move the plunger rod in the filling direction to fill the reservoir (draw up the drug delivery device with liquid drug, for example, from a vial or other drug container). The drive mechanism may be configured to be manually operated by a user requiring a user force or alternatively the drive mechanism may be adapted to be driven by an automatic drive unit such as an electric motor or electromagnetic actuator.

If the read information do not correspond to, match or is not in compliance with the predefined information the controller can lock the plunger rod and prevent a filling of the reservoir with drug and thus prevents the administration of the drug with the drug delivery device.

Such a mechanism can avoid administration errors such as the dispensing of an improper dose, a wrong drug or a dispensing at the wrong time or in inappropriate time interval. The comparison of the read information with predefined criteria thus may prevent an administration that is risky or even dangerous to the user's health.

The code reader is preferably adapted to read code information provided outside the delivery device on an external element, for example, on a drug package, a vial, a drug container or the like. The code reader may be implemented in form of an optical code reader or a code reader based on electromagnetic fields. Examples for optical codes are an Object Identifier code (OID), QR codes, bar codes, color codes and optical patterns. Examples for codes based on electromagnetic fields are a radio-frequency identification (RFID) tags or near-field communication (NFC) tags.

The read code information may be compared or verified with predefined information stored in a memory of the drug delivery device (previously transmitted to the delivery device) or received from an external device such as a user device, a cloud server or an external computer (received only at the time of comparison, after code reading). Data based on read code information may be uploaded to the external device for comparison with most up-to-date predefined data. The latter may be downloaded to the drug delivery device and stored. In this case the downloaded data include the predefined information.

If the controller compares the read information with the (received) predefined information the drug delivery device is preferably wirelessly connected to the external device allowing the exchange of data. The code information may include, for example, drug information such as drug type and name, expiry date of the drug and volume of the reservoir containing the drug.

The drive mechanism may be an automatic drive mechanism including an actuator to automatically move the plunger rod in a dispensing direction or in a filling direction opposite the dispensing direction. The actuator may be, for example, an electric motor or a solenoid actuator or an electric release mechanism configured to release a biased mechanical spring. Alternatively, the drive mechanism is a manual drive mechanism requiring a user force for moving the piston rod in dispensing and filling direction. In this case the controller controls, for example, a block mechanism with an electric blocking element blocking and thus preventing a manual movement of the plunger rod in the filing direction in case the read information are not in compliance with the predefined information. If the read information are in line with the predefined information the blocking mechanism releases the plunger rod such that a user can manually move the plunger rod in the dispensing or filling direction.

The plunger rod may form part of a reusable drive mechanism. In particular the delivery device may be implemented as a semi-disposable delivery device including a reusable assembly with the drive mechanism and a disposable assembly releasably attachable to the reusable assembly. The disposable assembly may be disposed of after a single use or if the reservoir is empty. In contrast, the reusable assembly may be used for a plurality of administrations with different disposable assemblies.

Alternatively, the plunger rod may be part of the disposable assembly. In this case the plunger rod is disposed of after use with the disposable assembly.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The predefined information includes preferably user-specific therapy data and the code information comprises drug data. After reading the code information the controller is adapted to extract the drug data and to compare the drug data with the user-specific therapy data stored in the delivery device or received from an external device. If the drug data match or are in compliance with the therapy data the controller may unlock the plunger rod such that the plunger rod can be moved from an initial or distal position to a proximal position allowing to draw up the delivery device and hence fill the reservoir of the delivery device.

Alternatively, the predefined information may comprise other data, such as for example manufacturer data (recall information, drug expire information) or delivery device data.

Preferably, the controller is configured to restrict the time period between the reading of the code information and a possibly enabling or disabling of a movement of the piston rod to a predefined time limit. That means the controller only enables or disables the movement within the predefined time frame, for example within several seconds after reading the code information on the drug container or drug package.

That means the controller is able to reduce the risk that user reads code information from a first drug and switches the device to a second drug and fills the reservoir with the second drug that is different from the first drug. Hence, the time limit set by the controller only allows to fill the reservoir with the correct drug providing the correct code information.

In a preferred embodiment the code reader is an optical code reader including an optical reader head oriented to or pointed to read a code outside the delivery device. That means the reader head points preferably away or at least parallel to a device longitudinal direction to be able to read an external code arranged on a drug package or a drug container (e.g. a vial). The code reader is thus preferably configured to read a code which is not inside the drug delivery device or not on a member of the drug delivery device.

Preferably, the plunger rod comprises a distal engagement member releasably connectable or engageable to the piston of the reservoir. The engagement member may be, for example, a snap fit, a thread or a spline connection. As the connection is a releasable connection the plunger rod can be detached or disconnected from the piston, for example, to exchange the reservoir or the connect the drive mechanism to another reservoir. This may be advantageous if the user intends to change the medication or if a different volume of the reservoir is required.

The piston comprises preferably an opening and the engagement member is preferably a flexible or elastic element (in particular a flexible arm) adapted to engage into the opening to connect the piston rod with the piston by a snap-fit connection. A snap-fit allows to reliably and easily connect the plunger rod with the piston.

The flexible element is preferably deflectable to connect and disconnect the plunger rod and the piston. In this case, if the plunger rod is driven towards the piston and abuts the piston the flexible element may be deflected and inserted into the opening and subsequently the flexible element may flex back into its initial position to establish the snap-fit connection. The connection between the plunger rod and the piston can therefore established only by moving the distal end of the piston rod into the opening in the piston.

The drug delivery device comprises preferably a release element with a contact surface adapted to deflect the flexible element when abutting the flexible element in a retracted positon or proximal end portion position of the plunger rod. The contact surface may be implemented as sloped or angled surface oriented in an acute angle relative to a plunger rod moving axis.

If the plunger rod is further moved in proximal direction the flexible element is forced to move out of an engaging position and thus the flexible element is released from the piston (release of snap-fit connection). Consequently, the plunger rod and the piston are disconnected. Disconnecting the plunger rod from the piston of the reservoir is advantageous in case the reservoir has to be exchanged. Due to the release element the flexible element can be easily and automatically deflected and thus released from the piston by moving the piston rod in proximal direction.

The drug delivery device preferably comprises an electric motor adapted to move the plunger rod in the dispensing direction (distal) or in the filling direction (proximal). The motor is preferably driven based on or according to the read code information. The read information in this case may comprise an instruction for drew up a dose (filling the reservoir with a specific amount of drug), the filling rate (motor speed and plunger rod speed for filing), a dispensing amount of dose, the delivery rate or a specific time for the dispensing. After reading the code information the controller operates the motor to fill the reservoir with liquid drug from a drug container (draw up) or to dispense the liquid drug from the reservoir.

The motor may be a stepper motor, a brushed or brushless DC motor or a synchronous motor.

In an alternative embodiment the device is rid of an electric actuator for moving the plunger rod. In this case the delivery device may comprises an electric actuator for automatically blocking, locking or unlocking the plunger rod and the filling movement and the dispensing movement has to be carried out manually by a force exerted by a user.

In a preferred embodiment the drug delivery device comprises a delivery device housing and a cover member movable relative to the device housing and relative to a dispensing end (distal end) of the drug delivery device between a cover position (distal position) and a retracted position (proximal position). In the cover position a dispensing end or a needle mounting portion of the drug delivery device is covered by the cover member and thereby a dispensing of the drug is prevented by the cover member. In the retracted position the dispensing end (or the needle mounting portion) is uncovered by the cover member and a dispensing of the drug is enabled as the dispensing end can be brought into contact with the injection site or infusion site of the user. The drug delivery device further comprises a locking member adapted to lock and fasten the cover member in the cover position. The controller is preferably configured to lock or unlock the cover member via the locking member based on the comparison of the read code information with the predefined information.

The controller may lock or unlock the cover member based on a verification or a match of the read information with predefined and stored or received data. A drug dispensing can thus only be carried out if the read information correspond to, match or are in compliance with the predefined information. In other words, if the read information does not correspond to, match to or is not in compliance with the predefined information the controller can lock the cover member in the cover position in order to prevent a dispensing of the drug because the cover member covers the dispensing end of the drug delivery device and thus a dispensing opening or a needle of the drug delivery device cannot be brought into contact with the injection site or the infusion site.

Such a mechanism can avoid an administration errors such as the dispensing of an improper dose, a wrong drug or a dispensing at the wrong time or in inappropriate time interval. The locking mechanism thus may prevent an administration that is risky or even dangerous to the user's health.

The cover member may be, for example, a cover sleeve, a cover sheet or a cover shield. The cover member is adapted to cover a dispensing end (distal end) or a possibly present needle mounting portion of the drug delivery device. The device does not mandatory have to have a needle. Needleless injectors are known in the art. Hence, the cover member may cover a possibly mounted needle, a dispensing opening or an injection end of a needleless drug delivery device. In the cover position a possibly mounted needle is covered by the cover member and thus the needle cannot be pierced or somehow brought into the user's skin and thus a dispensing of the drug is prevented. In contrast, in the retracted position the dispensing end, a dispensing opening or a needle mounting portion of the device is uncovered or revealed and thus a possibly mounted needle or a dispensing opening of the device can be brought into contact with the user allowing the administration of the drug.

The locking member provides a locking mechanism locking the cover member in the cover position. Locking means the cover member is not only hold but cannot be moved out of the cover position without a release movement of the locking member. The locking member may be, for example, a latching element, a hook, a spline or any member allowing a form fit locking or a locking with an electromagnet.

Preferably, the cover member can be locked or unlocked by a movement of the locking member relative to the delivery device housing and relative to the cover member. Hence, the locking member can be brought into engagement or disengagement with the cover member with a linear, rotational or helical movement or with a combination of such movements. The movement may open or release a form fit in order to allow the cover member to move between the cover position and the retracted position.

In a preferred embodiment the locking member is rotatable relative to the delivery device housing. This may in particular allow a space saving design.

The device preferably comprises an electric actuator adapted to move the locking member. The actuator may be an electric motor such as a servomotor or stepper motor or a linear or rotational electromagnetic solenoid actuator. The actuator is preferably separate or different than a possibly present actuator for moving the plunger rod. Based on the previous comparison of the read information with predefined information the controller operates the actuator to move locking member to lock or unlock the cover member.

Alternatively, the device comprises only a biased mechanical element such as a spring or an elastic member to move the locking member. In another embodiment the motor for moving the plunger rod may be used for moving the locking member.

In a preferred embodiment the locking member is a sleeve comprising on an inner surface a guiding structure or guiding means for guiding the cover member preferably in a linear direction. The cover member is thus guided by the locking member during its movement between the cover position and the retracted position. The locking member fulfils therefore two functions the locking and unlocking and the guiding of the locking member. The sleeve-shaped form of the locking member allows a compact design.

In an alternative embodiment the locking member may be implemented as a skirt, a tab or a hook preventing the cover member from moving in a locked state.

The locking member further preferably comprises an engagement member to releasably attach the reservoir to the delivery device housing. Examples for such an engagement member are a nut, a protrusion, a hook, a lug or a tab. Preferably the reservoir is held or attached to the delivery device housing by a spline engagement between the reservoir and locking member. The reservoir is thus preferably indirectly attached to the delivery device housing via locking member.

Preferably, the reservoir can be attached to the device housing by a rotational movement of the locking member relative to the device housing (e.g. a bayonet connection). By this movement of the locking member the engagement member is preferably brought into engagement with the reservoir and thus the engagement member holds the reservoir relative to the delivery device housing. In this attached state the drug delivery device can be used for filling the reservoir or for dispensing a dose.

The cover member is preferably sleeve-shaped and axially moveable relative to the device housing. The sleeve form allows a reliable covering of the dispensing end of the delivery device such that a dispensing is reliably prevented and a user cannot access the dispensing end if the cover member is in the cover position. Due to its axial relocatability the cover member can easily be switched between the cover position and the retracted position.

Alternatively, the cover member may be implemented as shield or skirt or the like.

The reservoir preferably comprises a spring adapted to bias the cover member in a distal direction and to bias the cover member into the cover position. That means the cover member is held by the spring in the cover position and can be moved only against the spring force away from the cover position. The spring ensures that the cover member covers the dispensing end of the drug delivery device in an initial state or if the drug delivery device is not used. If a user pushes the drug delivery device with its dispensing end (distal end) onto an injection site or an infusion site the cover member is moved against the spring force from the cover position towards the retracted position and thereby uncovers the dispensing end of the delivery device and revealing the needle (if any) or a dispensing opening of the device.

In a preferred embodiment the drug delivery device comprises a reservoir unit (or disposable unit or disposable assembly) comprising the reservoir and the piston and if present the cover member. The delivery device comprises further a drive unit (or reusable unit or reusable assembly) comprising the drive mechanism, the code reader and the controller and if present the locking member. The plunger rod may be either implemented in the reservoir unit (disposable plunger rod) or in the drive unit (reusable plunger rod). The reservoir unit is releasably attachable to the drive unit.

Such devices are named as reusable devices or semi-disposable devices as the reservoir unit may be replaced and disposed of after an injection or if the reservoir is empty. Subsequently, a new reservoir unit may be attached to the reusable drive unit in order to prepare the device for a new injection. The drive unit preferably comprises an electric motor as descripted above.

Alternatively, the drug delivery device may be an infusion device such as a drug pump or a patch pump.

The reservoir unit preferably comprises a receptacle or packaging enclosing the reservoir and the cover member in an initial or unused state. The receptacle protects the reservoir in such a state. Upon attachment of the reservoir unit the receptacle may be at least partially opened such that the reservoir unit can be attached to the drive unit. Before use of the delivery device the user has to remove the receptacle.

The invention further relates to a method for operating a drug delivery device. The method comprises the steps of
- reading, by a code reader of the drug delivery device, a code information of a drug;
- comparing, by a controller of the drug delivery device, the code information with predefined information,
- controlling a drive mechanism of the drug delivery device to block a piston rod of the delivery device or to move the piston rod in a dispensing direction to dispense a liquid drug from a reservoir or to move the piston rod in a filling direction opposite a dispensing direction to fill the reservoir with liquid drug based on the comparison.

The invention further comprises a method for dispensing a liquid drug with the drug delivery device. The method comprises the steps of
- Providing a drug delivery device with a code reader and a drug with a code including code information,
- Reading the code information by the code reader,
- Comparing the code information with predefined information,
- Unlocking a cover member of the delivery device, by a locking member, if the code information matches the predefined information,
- Retracting the cover member to uncover a dispensing end of the delivery device to enable an administration of the drug.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a reservoir unit and a drive unit of a first embodiment of an injection pen;
- Fig. 2: depicts a sectional view of the reservoir unit and the drive unit in an attached state;
- Fig. 3: depicts a sectional view of the drive unit;
- Fig. 4a,b: depict perspective views of a control sleeve;
- Fig. 5: depicts a perspective view of a reservoir unit without packaging;
- Fig. 6: depicts a sectional view of the injection pen during filling;
- Fig. 7: depicts a side view of the injection pen during scanning of a drug code;
- Fig. 8: depicts a side view of a second embodiment of an injection pen;
- Fig. 9: depicts a reservoir unit and a drive unit of the second embodiment;
- Fig. 10a,10b: depict a flow diagram of a use case with the injection pen.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side or end where an injection needle is attached. In contrast thereto the term "proximal" refers to the opposite side which is furthest away from the needle side or dispensing end.

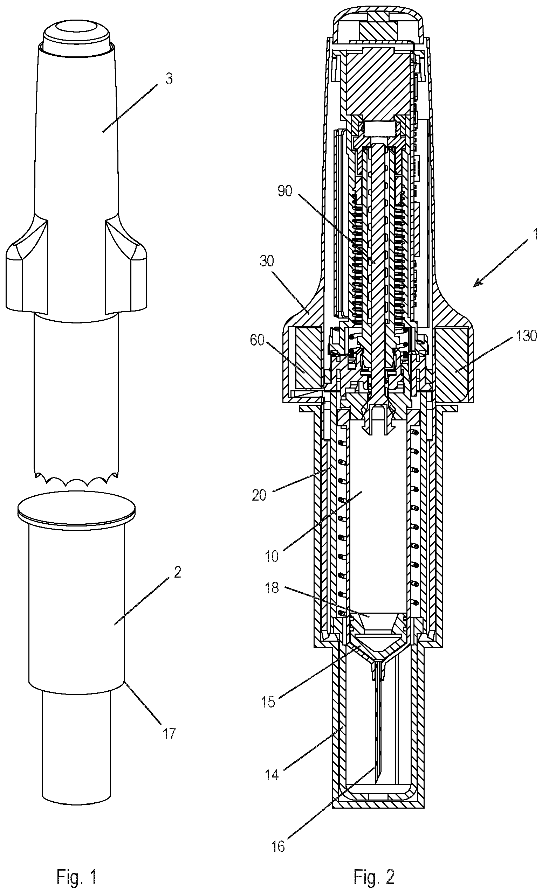

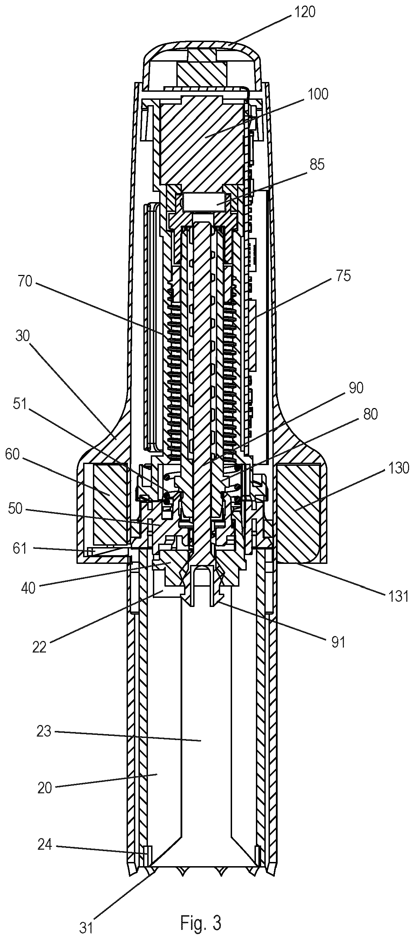

Figure 1 and 3 show a drug delivery device according to the invention. In the embodiment shown in the figures the device is implemented as a semi-disposable injection pen 1. The pen 1 comprises a disposable assembly in form of a reservoir unit 2 and a reusable assembly in form of a drive unit 3. The reservoir unit 2 is releasably attachable to the drive unit 3. In figure 1 the reservoir unit 2 is detached from the drive unit 3 and in figure 2 the reservoir unit is in an attached to the drive unit 3. Figure 2 depicts a sectional view of the injection pen 1 wherein the section runs along a longitudinal axis of the injection pen.

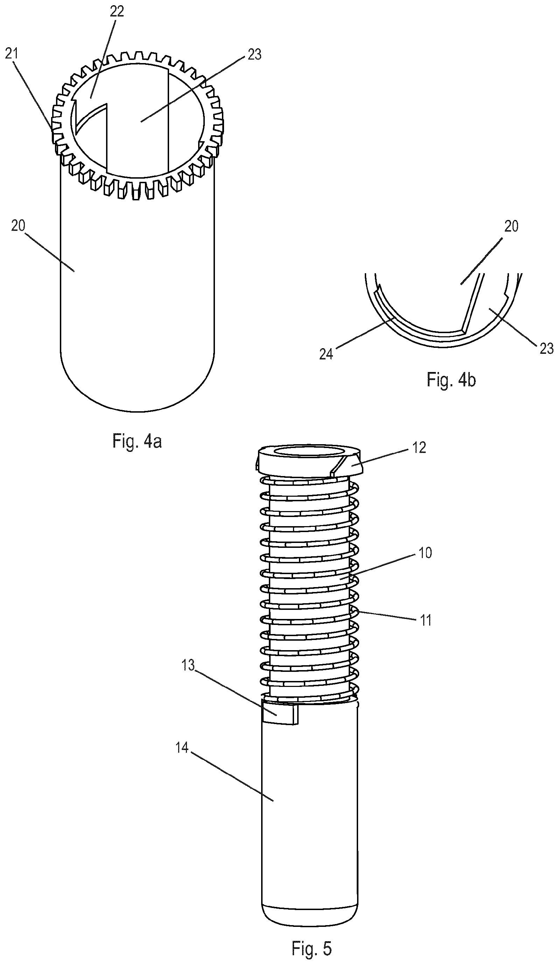

The reservoir unit 2 includes a reservoir 10 (figure 5) for a liquid drug, a needle shield 14 and a receptacle or packaging 17 (figure 1) encasing the reservoir 10 in an unused state. The packaging 17 comprises a foil sealing the reservoir 10. The drive unit 3 comprises a code reader 130 and a dose and dispensing mechanism with an electric motor 100. The reservoir unit 2 can be produced, pre-assembled and stored separately from the drive unit 3.

In the following the structural features of the injection pen 1 are descripted. Subsequently, the procedure to prepare and to carry out an injection with the injection pen will be descripted.

Figure 3 depicts a sectional view of the drive unit 3. The drive unit 3 comprises a cylinder-shaped housing 30 encasing the dose and dispensing mechanism. The housing 30 is provided with circumferentially arranged incisors 31 at a distal end of the housing 30. Inside the housing 30 in a distal portion a control sleeve 20 is arranged which is rotationally guided but axially fixed relative to the housing 30. The control sleeve 20 is described in detail with respect to figures 4a and 4b below. At a proximal end and radially offset of the control sleeve 20 an electric control sleeve motor 60 is located. Furthermore, a code reader 130 in form of an optical QR code reader with a reader head 131 is arranged radially offset at the proximal end. The control sleeve motor 60 and the code reader 130 are circumferentially spaced apart from each other as it can be seen in figure 2.

On a proximal side of the control sleeve 20 is a contact member 40 arranged and proximal to the contact member 40 an insert 50 is located inside the housing 30. The insert 50 is rotationally fixed and axially moveable with respect to the housing 30. A coupling spring 51 is arranged between a proximal surface of the insert 50 and a distal surface of a sleeve-shaped mechanics holder 70 thus biasing the insert 50 in a distal direction towards the control sleeve 20. The mechanics holder 70 is rotationally and axially non-movable held by the housing 30. A printed circuit board (PCB) 75 including a controller (not shown) and a motor drive circuit (not shown) is radially located between an outside of the mechanics holder 70 and inside an inner wall of the housing 30.

A drive sleeve 80 is coaxially arranged inside the mechanics holder 70 and axially fixed but rotatably guided by the insert 50. The drive sleeve 80 comprises a nut on its inside which is splined to a plunger rod 90 being located coaxially inside the drive sleeve 80. As the plunger rod 90 is in threaded connection with the insert 50 a rotation of the drive sleeve 80 causes the plunger rod 90 to screw in distal or proximal direction, respectively. At its proximal end portion the drive sleeve 80 is rotationally connected to a gear 85 which in turn is connected to a motor shaft of the electric motor 100 rotationally and axially non-movable held by the mechanics holder 70 inside the housing 30. At a proximal end of the housing 30 a push button 120 is arranged that can be pushed by the user in distal direction in order to initiate an injection.

Figure 4a depicts a perspective view of the control sleeve 20. As shown in figure 4a the control sleeve 20 is provided with circumferentially arranged teeth 21 at a proximal end of the control sleeve 20. These teeth 21 engage counter teeth of a toothed drive wheel 61 of the control sleeve motor 60. Hence, if the control sleeve motor 60 is activated the motor drive wheel 61 rotates and thus the control sleeve 20 rotates relative to the housing 30 around the longitudinal axis of the injection pen 1. The control sleeve motor 60 is powered by an energy source in form of a rechargeable battery inside the housing (not shown).

On its inside the control sleeve 20 is further provided with a longitudinal nut 23. At a proximal end portion the control sleeve 20 comprises on its inside a circumferential proximal nut 22 and at its distal end portion the sleeve has a circumferential distal nut 24 shown in figure 4b which depicts a sectional view of the control sleeve 20.

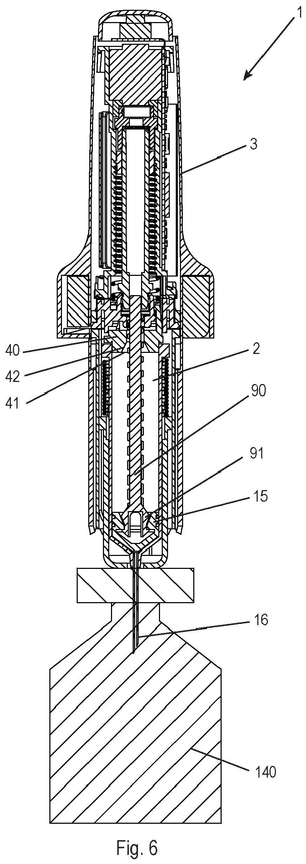

Figure 5 depicts a perspective view of the reservoir unit 2 without the encasing packaging 17. As shown the reservoir unit 2 comprises a cylindrical reservoir 10 having a piston 15 inside (shown in figure 2 and 6), a sleeve-shaped needle shield 14 movable in an axial direction relative to the reservoir 10 and a needle shield spring 11 biasing the needle shield 14 in a distal direction. The needle shield 14 is movable relative to the reservoir 10 between a distal cover position and a proximal retracted position. In the retracted position a needle 16 (figure 6) is uncovered whereas in the cover position the needle shield 14 completely covers the needle 16. The reservoir 10 comprises on its proximal end a flange with two radially protruding reservoir cams 12 circumferentially spaced apart each other and adapted to engage with the proximal nut 22 of the control sleeve 20. Additionally, the needle shield 14 comprises at its proximal end two radially protruding shield cams 13 adapted to engage the longitudinal nut 23 or the distal nut 24 of the control sleeve 20.

Figure 6 depicts a sectional view of the injection pen 1 with the needle shield 14 in the retracted position. The reservoir unit 2 is attached to the drive unit 3 and the distal end portion of the plunger rod 90 is connected to the piston 15 inside the reservoir 10 by a snap-fit connection. For that purpose, the distal end portion of the plunger rod 90 comprises two flexible and elastic arms 91 adapted to be deflected radially inward if the distal portion is inserted into an opening 18 centrally arranged in the piston 15.

In the following an injection process with the injection pen 1 according to the invention is described.

Before an injection the user attaches the reservoir unit 2 to the drive unit 3. That is, the user pierces with the distal arranged incisors 31 of the housing 30 of the drive unit 3 the proximal protective foil of the packaging 17 of the reservoir unit 2. Alternatively, the user pulls off manually the foil before use. The user then inserts a distal housing portion inside the packaging 17 such that the housing 30 and the control sleeve 20 are radially in-between an inner cylindrical wall of the packaging 17 and an outer wall of the reservoir 10. During this movement the reservoir cam 12 are inserted and travel along the longitudinal nut 23 of the control sleeve 20. If an end position is reached the proximal end wall of the reservoir 10 abuts or physically contacts a distal wall of the contact member 40. The contact member 40 is thus little displaced against the coupling spring 51 in proximal direction. This movement is detected by a sensor circuit (not shown) which wakes up a control circuit from a power save mode. The controller is now aware that the user intends to couple the reservoir unit 2 with the drive unit 3 and drives the control sleeve motor 60. The sleeve motor 60 rotates the control sleeve 20 a short distance (about 15°) in a first direction relative to the housing 30 and relative to the reservoir 10. By this movement the reservoir cams 12 enter the proximal nut 22 of the control sleeve 20. The reservoir unit 2 is then locked to the drive unit 3 by a form fit connection. In other words, the rotation of the control sleeve 20 relative to the reservoir 10 couples the reservoir unit 2 to the drive unit 3 (similar to a bayonet connection). The shield cams 13 of the needle shield 14 are in the distal nut 24 of the control sleeve 20 and thus prevent the needle shield from being movable in proximal direction. Hence, in this initial or locked state the needle shield 14 is locked by the control sleeve 20 in the cover position.

The controller of the PCB of the drive unit receives delivery instructions from the code reader 130 or from an external device via wireless data transmission. Based on the received data the controller drives the motor 100 to prepare an injection.

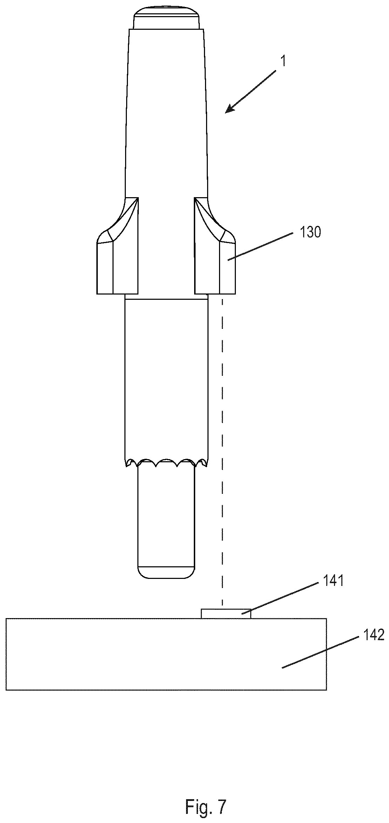

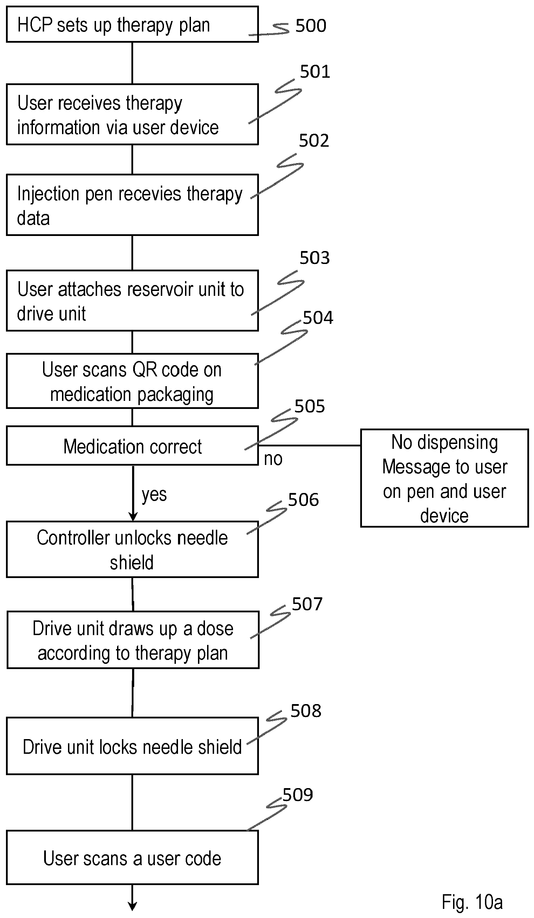

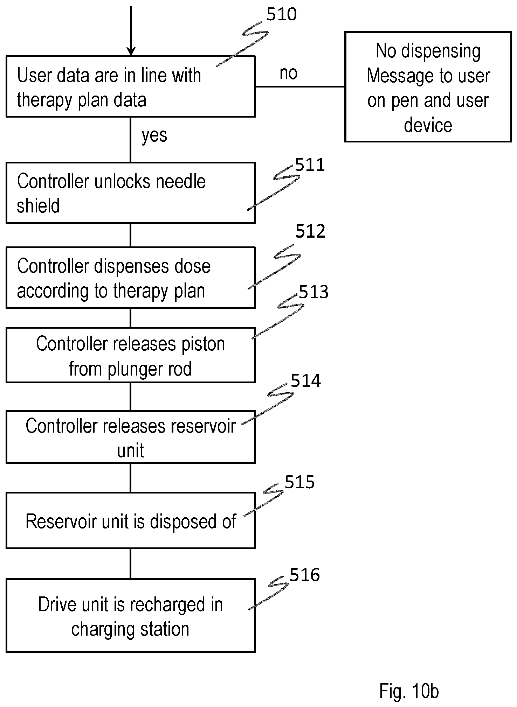

With the reader head 131 of the code reader 130 of the drive unit 3 the user scans a QR code 141 or any optical pattern located on a drug packaging 142 or on a drug container (e. g. vial 140). As shown in figure 7 the code reader head 131 points towards the external code 141 and catches the optical code 141 on drug packaging 142. The controller receives the read information and compares the extracted data with predefined therapy data. An exemplary use case is described further below (figures 10a and 10b). In case the read code data are in compliance with the predefined criteria the controller allows an injection.

For this purpose, the controller drives the control sleeve motor 60 to rotate the control sleeve 20 again in the first direction relative to the housing 30 and relative to the reservoir 10. This causes the shield cams 13 to come out of the distal nut 24 of the control sleeve 20 and being rotationally positioned in line with the longitudinal nut 23. As the shield cams 13 can now enter the longitudinal nut 23 the needle shield 14 can be moved against a force of the needle shield spring 11 from the cover position in proximal direction into a retracted position.

The controller operates the motor 100 in the drive unit 3 to move the plunger rod 90 in a distal direction towards the piston 15 inside the reservoir 10. Before a distal end position is reached the flexible arms 91 of plunger rod 90 are brought into contact with the opening 18 or hole of the piston 15. As the opening 18 has a conical form the flexible arms 91 are forced to deflect radially inward to the center of the device and thus the arms 91 enter into the opening. After the arms have passed a narrow section inside the opening they can move radially back outward and thus enable a form fit connection with the piston 15.

After reaching this distal end position the controller drives the motor 100 to move the plunger rod 90 back in proximal direction. As the plunger rod 90 is connected to the piston 15 the piston is moved proximally too and a liquid drug can be drawn up from a drug container, e. g. a vial 140 as shown in figure 6. The controller received the information for the amount of the dose to be prepared from a user-specific code previously read by the code reader.

After a dose of drug has been drawn up and filled into reservoir 10 the controller stops the motor 100 and the user takes the injection pen 1 away from the vial 140. As the needle shield 14 is biased by the spring 11 towards the cover position the needle shield 14 moves distally into the cover positon as soon as the injection pen is moved away from the vial 140. Subsequently, the controller drives the control sleeve motor 60 to rotate the control sleeve 20 in a second direction opposite the first direction. That causes the shield cams 13 to enter again into the distal nut 24 of the control sleeve 20 and thus locking the needle shield 14 in the cover position.

The controller may rotate the control sleeve 20 again in the first direction to unlock the needle shield 14 after the controller as received an approval for an injection. This may be the case, if the code reader 130 has read the optical code 141 associated with a user and if the read code information is in compliance with therapy information. After unlocking the needle shield 14 and after the user has placed the injection pen 1 onto the injection site the controller drives the motor 100 to move to plunger rod 90 with the piston 15 in the distal direction and thereby dispensing the drug from the reservoir 10.

After the injection process controller rotates the control sleeve 20 again in the second direction to lock the needle shield 14. An injury with the injection needle may thus be prevented. Subsequently, the motor 100 moves the plunger rod 90 back in the proximal direction. Before the plunger rod 90 reaches the proximal end position an outer sides of the flexible arms 91 of the plunger rod abut a contact surface 42 of a conical opening 41 in the contact member 40 (shown in figure 6). As the plunger rod 90 is further moved in proximal direction the two flexible arms 91 are forced to move radially inward due to the conical form of the opening 41 in the contact member 40. By this inward movement the arms 91 release the snap fit connection and thus are released from the piston 15. The plunger rod 90 is thus no longer connected to the piston 15 of the reservoir.

In a further step the controller drives the control sleeve motor 60 to rotate the control sleeve 20 in the second direction opposite the first direction until the reservoir cam 12 come out of the proximal nut 22 of the control sleeve. The connection between the reservoir unit 2 and the drive unit 3 is therefore released and the reservoir unit 2 can be detached from the drive unit 3.

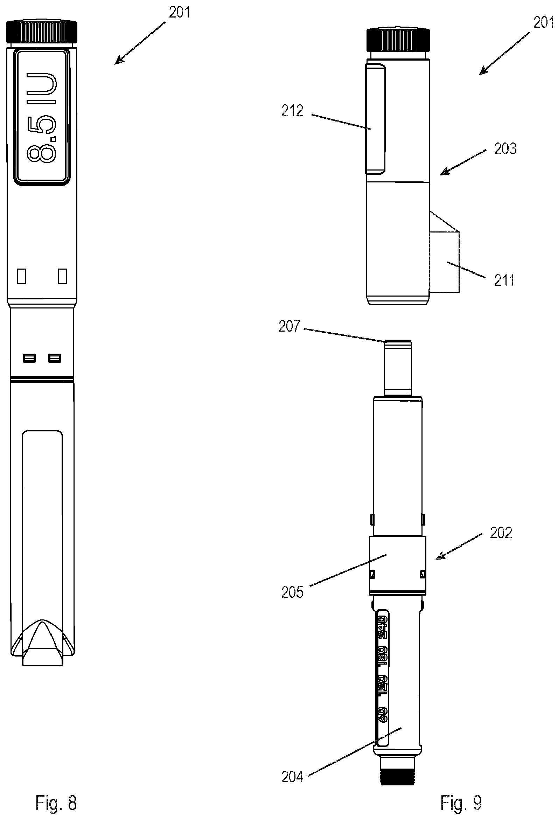

Figures 8 and 9 depict a second embodiment of a drug delivery device according to the invention. In this second embodiment the drug delivery device is implemented as a semi-disposable injection pen 201. The pen 201 comprises a disposable assembly or reservoir unit 202 which is disposed off after use and a reusable assembly 203 which is used for a plurality of injections.

Figure 9 depicts the injection pen 201 of figure 8 wherein the reservoir unit 202 is detached from the reusable assembly 203. The reservoir unit 202 includes a reservoir holder 204 (or cartridge holder), a reservoir in form of a cartridge (not shown) adapted to contain the liquid drug, a housing 205 and a plunger rod connected to a piston inside the cartridge (not shown).

The housing 205 is non-dissociable connected to the reservoir holder 204 by a snap lock. The reservoir holder 204 is thus fixed relative to the housing 205 and encloses and holds the cartridge non-movable. Inside the housing 205 a plunger rod driver 207 is coaxially arranged and guided. The plunger rod driver 207 is splined to the plunger rod (not shown). At the proximal end the plunger rod driver 207 comprises circumferentially arranged teeth adapted to be rotationally coupled to a drive member (not shown) of an automatic drive mechanism in the reusable assembly 203.

The reservoir unit 202 can be produced, pre-assembled and stored separately from the reusable assembly 203. Single parts of the reservoir unit 202 may be produced by a device manufacturer and delivered to the drug or medication manufacturer. The latter inserts the filled cartridge in the reservoir holder 204 and pre-assembles the single components to the sub-assembled reservoir unit 202.

The reusable assembly 203 includes a code reader 211 and the automatic drive mechanism with an electric motor (not shown) adapted to rotate the drive member and thus the plunger rod, if the reservoir unit 202 is attached to the reusable assembly 203. The latter comprises further a controller, a data storage module, a data transmission module (not shown) and display 212. The reusable assembly 203 is releasably attachable to the cartridge unit 202.

The function of the injection pen 201 of the second embodiment is similar to the injection pen according to the first embodiment. In contrast to the first embodiment the reservoir unit 202 of the second embodiment includes the plunger rod. That means the plunger rod is inside the pre-assembled reservoir unit 202 and is permanently connected to the piston inside the cartridge. Furthermore the injection pen 201 in the second embodiment does not comprise a needle shield, a control sleeve and a control sleeve motor. Furthermore, the injection pen 201 does not comprise a pre-assembled injection needle. The user mounts a needle on a distal needle mounting portion before an injection. All other function described above apply also for the second embodiment, in particular the function relating to the code reader 211.

In a third embodiment the injection pen does not comprise an electric motor. Instead, the drive mechanism is implemented as a manual dose and dispensing mechanism requiring the user to manually set a dose by turning a dose sleeve and requiring a manual force to inject the set dose. Such dose and dispensing mechanism are known in the art and disclosed, for example, in the patent application WO20187547A1 which is hereby incorporated by reference. Except the manual dose and dispensing mechanism the third embodiment of the injection pen comprises all features described above with respect to the first embodiment. In particular, the injection pen according to the third embodiment also comprises the control sleeve, the control sleeve motor, the code reader and the plunger rod with the arms for engaging the piston inside the reservoir. Hence, the third embodiment also comprises the same reservoir unit with the reservoir and the needle shield as descripted above with respect to the first embodiment

In the following a possible use case is described in detail with reference to figures 10a and 10b. The injection process may be carried out by the user at home or in a hospital or by a health care practitioner (HCP) in a healthcare environment.

In a first step 500 the HCP sets up a therapy plan defining, for example, the medication, the amount of doses to be administered and a timetable for the administrations. The HCP allocates the defined therapy plan to a user. In a second step 501 the user or another HCP receives the therapy plan data on a user device or HCP computing device which sends the therapy data to the injection pen (e.g. via Long Range Area Network, mobile network, Bluetooth) in step 502. Alternatively, the injection pen receives the therapy data directly from a cloud server.

In order to prepare the injection the user or the HCP attaches the reservoir unit 2 to the drive unit 3 (as described in detail above) in step 503. The controller of the injection pen 1 drives the control sleeve motor 60 to fasten the reservoir unit 2 to the drive unit 3 by the control sleeve 20. The controller further drives the motor 100 for moving the plunger rod 90 towards the piston 15 to couple the plunger rod 90 with the piston 15.

Subsequently, the user or HCP scans a QR code on the packaging of the medication with the code reader 130 of the injection pen 1 in step 504. The controller in the drive unit processes the read information and verifies the data from the read QR code with the therapy data (Step 505). If the read medication data does not match the therapy data (e.g. if the chosen medication not appropriate or if the time for an injection is not appropriate) and/or if the time limit before starting the filling process is exceeded the controller disables the dispensing mechanism of the injection pen and signals it to the user by a red LED of the injection pen. The user or HCP may additionally or instead receive a notification on the user or HCP device. In case the injection pen comprises a display the error or message is displayed on the pen display.

If the medication corresponds to the predefined therapy data and/or if the time for the injection is in line with the therapy plan and if the time limit after reading the code is not exceeded the controller unlocks the needle shield 14 by the control sleeve 20 (step 506) such that it can move from the cover position to the retracted position. The controller detects the retracted position of the needle shield by means of a micro switch. The user or HCP holds the uncovered needle in a vial 140 and confirms this by pressing the button 120 on the injection pen. In step 507 the motor is controlled to move the plunger rod back in proximal direction in order to fill the reservoir 10 with the liquid drug (draw up medication) if the needle shield 14 is detected to be in the retracted position. The filling is carried out according to the therapy data.

If a predefined amount of drug is inside the reservoir 10 according to the therapy plan the motor 100 stops the plunger rod 90 and locks the needle shield 14 by the control sleeve 20 in the cover position as soon as the user has retracted the injection pen from the vial 140 in step 508.

In step 509 the user or HCP scans with the code reader 130 of the injection pen a code associated with the user (e. g. a user bracelet or the like including a QR code). The controller in the drive unit verifies the read user information with the therapy data (step 510). If the data do not match the controller locks the dispensing mechanism of the injection pen and indicates (LED, notification on user device) that the user does not correspond to the therapy data. If the read user information are in line with the therapy data the controller unlocks the needle shield 14 by the control sleeve 20 in step 511. Consequently, the user or HCP places the injection pen onto the injection side and confirms it by pressing the button. In step 512 the controller drives the motor 100 to dispense the dose according to the therapy plan if the needle shield 14 is detected to be in the retracted position (micro switch).

If the dose is dispensed and if the user has removed the injection pen from the injection site the motor moves back the plunger rod 90 towards a proximal end position where the plunger rod 90 is uncoupled from piston 15 of the reservoir 10 (step 513). The controller further drives the control sleeve motor 60 to uncouple or release the reservoir unit 2 from the drive unit 3 in step 514. Consequently, the user or HCP can detach the reservoir unit 2 from the drive unit 3 and can dispose of the reservoir unit 2 in step 515. The researchable battery of the drive unit may be charged if necessary (step 516) in a charging station. In a hospital environment the charging station can be implemented in a wearable, for example, in a belt of the HCP.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | Injection pen | 60 | control sleeve motor |
| 2 | Reservoir unit | 61 | drive wheel |
| 3 | drive unit | 70 | mechanics holder |
| 10 | reservoir | 75 | PCB |
| 11 | spring | 80 | drive sleeve |
| 12 | reservoir cam | 85 | gear |
| 13 | shield cam | 90 | plunger rod |
| 14 | needle shield | 91 | arms |
| 15 | piston | 100 | motor |
| 16 | needle | 120 | button |
| 17 | packaging | 130 | code reader |
| 18 | opening | 131 | reader head |
| 20 | control sleeve | 140 | vial |
| 21 | teeth | 141 | optical code |
| 22 | proximal nut | 142 | drug packaging |
| 23 | longitudinal nut | | |
| 24 | distal nut | 201 | injection pen |
| 30 | housing | 202 | reservoir unit |
| 31 | incisors | 203 | reusable assembly |
| 40 | contact member | 204 | reservoir holder |
| 41 | conical opening | 205 | housing |
| 42 | contact surface | 207 | plunger rod driver |
| 50 | insert | 211 | code reader |
| 51 | coupling spring | 212 | display |

## Claims

1. A drug delivery device (1) for dispensing a liquid drug from a reservoir (10), the device comprising
- a plunger rod (90);
- a drive mechanism for moving the plunger rod (90) in a dispensing direction and in a filling direction opposite the dispensing direction;
- the reservoir (10) including a piston (15) movable relative to a reservoir outlet,
wherein the plunger rod (90) is connectable to the piston (15) to move the piston (15) in both the dispensing direction and the filling direction,
**characterized in that**
the delivery device (1) further comprises a code reader (130) for reading code information and a controller adapted to compare the read code information with predefined information and adapted to control the drive mechanism to block the piston rod (90) or to enable movement of the piston rod (90) in the filling direction to fill the reservoir (10) with liquid drug based on the comparison.

2. Drug delivery device (1) according to claim 1, wherein the predefined information comprises a user-specific therapy information and the code information comprises drug data.

3. Drug delivery device (1) according to claim 1 or 2, wherein the controller is configured to restrict the time period between the reading of the code information and an enabling of movement of the piston rod to a predefined time limit.

4. Drug delivery device (1) according to any of claims 1 to 3, wherein the code reader (130) is an optical code reader including an optical reader head (131) arranged to read a code (141) outside the delivery device (1), in particular on a drug container (140) adjacent to a reservoir outlet, wherein the orientation of the reader head (131) points to the code information when a drug container inlet is placed next to the reservoir outlet.

5. Drug delivery device (1) according to any of claims 1 to 4, wherein the plunger rod (90) comprises a distal engagement member releasably connectable to the piston (15) of the reservoir (10).

6. Drug delivery device (1) according to claim 5, wherein the piston (15) comprises an opening (18) and the engagement member is a flexible element (91) adapted to be inserted into the opening (18) to connect the piston rod (90) with the piston (15) by a snap-fit connection.

7. Drug delivery device (1) according to claim 6, wherein the delivery device (1) comprises a release element (40) with a contact surface (42) adapted to deflect the flexible element (91) when abutting the flexible element (91) in a retracted positon of the plunger rod (90).

8. Drug delivery device (1) according to any of claims 1 to 7, comprising a device housing (30) and a cover member (14) movable relative to the device housing (30) between a cover position and a retracted position, wherein
- in the cover position a dispensing end of the drug delivery device (1) is covered by the cover member (14) and thereby an administering of the drug is prevented and
- in the retracted position the dispensing end is uncovered and a dispensing of the drug is enabled;
wherein the drug delivery device (1) further comprises a locking member (20) adapted to lock the cover member (14) in the cover position,
wherein the controller is adapted to lock or unlock the cover member (14) via the locking member (20) based on the comparison of the read code information with predefined information.

9. Drug delivery device (1) according to claim 8, wherein the cover member (14) can be locked or unlocked by a movement, preferably by a rotational movement, of the locking member (20) relative to the device housing (30).

10. Drug delivery device (1) according to claim 9, wherein the device comprises an electric actuator (60) adapted to move the locking member (20).

11. Drug delivery device (1) according to any of claims 8 to 10, wherein the locking member (20) is a sleeve comprising on an inner surface a guiding structure (23, 24) for guiding the cover member (14).

12. Drug delivery device (1) according to any of claims 8 to 11, wherein locking member (20) further comprises an engagement member to releasably attach the reservoir (10) to the delivery device housing (30).

13. Drug delivery device (1) according to any of claims 8 to 12, wherein the drug delivery device (1) further comprises a spring (11) adapted to bias the cover member (14) in a distal direction and in the cover position.

14. Drug delivery device (1) according to any of claims 1 to 13, wherein the device comprises a
- a reservoir unit (2) comprising the reservoir (10) with the piston (15) and
- a drive unit (3) comprising the drive mechanism, the code reader (130) and the controller; wherein the reservoir unit (2) is releasably attachable to the drive unit (3).

15. Method for operating a drug delivery device, the method comprising the steps of
- Providing a drug delivery device (1) with a code reader (130) and a drug with a code (151) including code information,
- Comparing, by a controller of the drug delivery device, the code information with predefined information,
- control a drive mechanism of the drug delivery device to block a piston rod of the delivery device or to move the piston rod in a filling direction opposite a dispensing direction to fill the reservoir with liquid drug based on the comparison.
